(19) **Europäisches Patentamt**
**European Patent Office**
**Office européen des brevets**

(11) Publication number : **0 346 075 B1**

(12) # EUROPEAN PATENT SPECIFICATION

(45) Date of publication of patent specification :
**02.01.92 Bulletin 92/01**

(51) Int. Cl.[5] : **A61K 31/43,** A61K 9/06,
A61K 47/00

(21) Application number : **89305702.6**

(22) Date of filing : **06.06.89**

(54) **Veterinary composition.**

The file contains technical information
submitted after the application was filed and
not included in this specification

(30) Priority : **09.06.88 GB 8813670**

(43) Date of publication of application :
**13.12.89 Bulletin 89/50**

(45) Publication of the grant of the patent :
**02.01.92 Bulletin 92/01**

(84) Designated Contracting States :
**AT BE CH DE ES FR GB GR IT LI LU NL SE**

(56) References cited :
**EP-A- 0 271 306**
**FR-A- 2 320 749**
**GB-A- 2 119 649**
**Remingtons Pharm. Sci. 1975 p.1128-1129**

(73) Proprietor : **BEECHAM GROUP PLC**
**Beecham House Great West Road**
**Brentford Middlesex TW8 9BD (GB)**

(72) Inventor : **Dowrick, John Sidney Beecham**
**Parmaceuticals**
**Walton Oaks Dorking Road**
**Tadworth Surrey KT20 7NT (GB)**

(74) Representative : **West, Vivien et al**
**Beecham Pharmaceuticals Great Burgh Yew**
**Tree Bottom Road**
**Epsom Surrey KT18 5XQ (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

GB-A-2 119 649 describes a method for the treatment of infectious keratoconjunctivitis in animals, which comprises administering to the animal by topical instillation, a formulation comprising a salt of cloxacillin and a veterinarily acceptable oil as carrier. The method is especially applicable in the treatment of Infectious Bovine Keratoconjunctivitis.

The invention provides the use of a formulation comprising ampicillin or a veterinarily acceptable salt thereof, a veterinarily acceptable salt of cloxacillin, and a veterinarily acceptable oil as carrier, for the manufacture of a medicament for topical use in the treatment or prophylaxis of eye infections in animals.

The formulations of the invention provide prolonged inhibitory levels of antibiotic and are useful in the treatment of eye infections in animals caused by a wide range of organisms.

As used herein the term "eye infection" includes but is not limited to conjunctivitis, such as infectious keratoconjunctivitis.

The ampicillin is typically present as the trihydrate and comprises 3 to 10% w/w, preferably 5% w/w, of the formulation (calculated as the free acid).

The formulations suitably contain sodium or benzathine cloxacillin. Typically, the concentration of cloxacillin salt is from 5 to 20% w/w, preferably 10% of the formulation (calculated as the free acid). Suitably, the oil base is a mineral oil base, preferably liquid paraffin gelled with from 0 to 5% by weight of the base of aluminium stearate, or a mixture of soft paraffin and liquid paraffin, or a vegetable oil such as arachis oil.

The formulation may additionally contain pain relieving agents, antiinflammatory agents such as corticosteroids, and the like. Preferably, the formulation comprises up to 0.5%, more particularly 0.25%, prednisolone.

Preferably the penicillins are present in the form of finely divided particles, as described in EP-A-0271 306 (USSN 129,989).

Advantageously, the penicillins are administered in the form of particles, at least 65% by weight of which have a size in the range 0-5 μ, suspended in a hydrophobic oily vehicle which comprises an oil and a gelling agent.

Advantageously at least 94% of the particles have a size in the range 0-13 μ. Preferably less than 55% of the particles are larger than 3 μ. The particles may be prepared by techniques including bead-milling, and micronization. The use of bead milling is particularly preferred, as it obviates the need for handling the finely divided penicillins in dry powder form.

Suitable vehicles are as set out above.

A number of known containers suitable for instillation of the formulation into an infected eye may be used. Preferably, the container is a sealable aluminium tube, or a polyethylene syringe.

The dosage regimes will vary according to the size of the sufferer. A suitable dosage unit is generally between 20 to 250 mg of penicillin per eye. For the treatment of cattle, sheep, goats and horses a dose of about 100 mg of penicillin per eye is appropriate. A smaller dose, such as about 50 mg per eye, is suitable in the treatment of domestic animals, such as dogs or cats.

The treatment may require a single application of the formulation, or severe infections may require more than one application.

Veterinary formulations for use in the present invention may be prepared by mixing the penicillins with the vehicle and with any other components of the formulation. The process may suitably be carried out as follows:-

(a)    the oil is mixed with the gelling or thickening agent, and the mixture is heat sterilized and allowed to cool,

(b)    the powdered active ingredient is mixed into the base with stirring,

(c)    high shear mixing equipment is used to produce a fine homogenous dispersion, and

(d)    the mixture is subjected to bead-milling.

The formulation is then packed in an appropriate form for administration, eg. syringe or tube.

The following Examples illustrate the invention.

Example 1

```
                                           %  w/w
      Ampicillin Trihydrate ........... 5.00 (as free acid)
      Benzathine Cloxacillin........... 10.00 (as free acid)
      Base......................... to 100.00
```

Base composition

```
      Aluminium Stearate................ 3.00
      Liquid Paraffin...............to 100.00
```

The base was prepared by heating together and sterilising the aluminium stearate and liquid paraffin. When cool the sterile antibiotics were added and incorporated by high shear mixing. The mixture was then passed through a rotary bead mill under the following conditions :

| | |
|---|---|
| Equipment : | 'Dyno-Mill' Model KDL Pilot |
| Zirconium beads : | 1.0-1.25 mm |
| Gap setting : | 0.3 mm |
| Feed rate : | 4.2 litres/hour |
| Rotor speed : | 2400 RPM |

Part of the finished suspension was filled as 3 g doses into lacquered aluminium tubes.

Example 2

```
                                           %  w/w
      Ampicillin Trihydrate............. 5.00 (as free acid)
      Benzathine Cloxacillin.............10.00 (as free acid)
      Prednisolone........................ 0.25
      Base......................... to 100.00
```

Base composition

```
      Aluminium stearate................  3.00
      Liquid Paraffin................to 100.00
```

The process was carried out as in Example 1, except that 0.25% prednisolone was added to the base immediately prior to the antibiotic agents, by admixture with a small portion of base which was then added to the bulk of the base.

**Claims**

1. The use of a formulation comprising ampicillin or a veterinarily acceptable salt thereof, a veterinarily acceptable salt of cloxacillin, and a veterinarily acceptable oil as carrier, for the manufacture of a medicament for topical use in the treatment or prophylaxis of eye infections in animals.

2. Use according to claim 1, wherein the ampicillin is present as the trihydrate and comprises 3 to 10% w/w of the formulation.

3. Use according to claim 1 or 2, wherein the cloxacillin is present as the sodium or benzathine salt and comprises from 5 to 20% w/w of the formulation.

4. Use according to any one of claims 1 to 3, wherein the veterinarily acceptable oil is a mineral oil.

5. Use according to any preceding claim, wherein the formulation additionally comprises one or more pain relieving agents and/or antiinflammatory agents.

6. Use according to claim 5, wherein the formulation comprises up to 0.5% prednisolone.

7. Use according to any preceding claim, wherein the penicillins are present in the form of finely divided particles, at least 65% by weight of which have a size in the range 0-5 μ, suspended in a hydrophobic oily vehicle which comprises an oil and a gelling agent.

8. Use according to claim 7, wherein at least 94% of the particles have a size in the range 0-13 μ.

9. Use according to claim 7 or 8, wherein the particles are prepared by bead-milling.

10. Use according to any one of the preceding claims, wherein the formulation is in unit dosage form containing not more than 250 mg of penicillin per unit dose.

**Patentansprüche**

1. Verwendung einer Formulierung, umfassend Ampicillin oder ein veterinärverträgliches Salz davon, ein veterinärverträgliches Salz von Cloxacillin und ein veterinärverträgliches Öl als Träger, zur Herstellung eines Arzneimittels zur örtlichen Verwendung bei der Behandlung oder Prophylaxe von Augeninfekten bei Tieren.

2. Verwendung nach Anspruch 1, wobei das Ampicillin als Trihydrat vorliegt und 3 bis 10 % Gew./Gew. der Formulierung umfaßt.

3. Verwendung nach Anspruch 1 oder 2, wobei das Cloxacillin als Natrium oder Benzathinsalz vorliegt und 5 bis 20 % Gew./Gew. der Formulierung umfaßt.

4. Verwendung nach einem der Ansprüche 1 bis 3, wobei das veterinärverträgliche Öl ein Mineralöl ist.

5. Verwendung nach einem vorangehenden Anspruch, wobei die Formulierung zusätzlich ein oder mehrere schmerzlindernde Mittel oder entzündungshemmende Mittel umfaßt.

6. Verwendung nach Anspruch 5, wobei die Formulierung bis zu 0, 5 % Prednisolon umfaßt.

7. Verwendung nach einem vorangehenden Anspruch, wobei die Penicilline in Form von feinverteilten Teilchen vorliegen, von denen mindestens 65 Gew.-% eine Größe im Bereich von 0 bis 5 μ besitzen, die in einem hydrophoben Ölträger suspendiert sind, der ein Öl und ein Gelierungsmittel umfaßt.

8. Verwendung nach Anspruch 7, wobei mindestens 94 % der Teilchen eine Größe im Bereich von 0 bis 13 μ besitzen.

9. Verwendung nach Anspruch 7 oder 8, wobei die Teilchen mit einer KugelmühLe hergestellt wurden.

10. Verwendung nach einem der vorangehenden Ansprüche, wobei die Formulierung in Einheitsdosierform vorliegt, die nicht mehr als 250 mg Penicillin je Einheitsdosis enthält.

**Revendications**

1. Utilisation d'une formulation comprenant de l'ampicilline ou un sel de celle-ci acceptable du point de vue vétérinaire, un sel acceptable du point de vue vétérinaire de cloxacilline et une huile acceptable du point de vue vétérinaire en tant que support, pour la fabrication d'un médicament à usage topique dans le traitement ou la prophylaxie d'infections ophtalmiques chez les animaux.

2. Utilisation suivant la revendication 1, caractérisée en ce que l'ampicilline est présente sous forme du trihydrate et représente 3 à 10 % en p/p de la formulation.

3. Utilisation suivant la revendication 1 ou la revendication 2, caractérisée en ce que la cloxacilline est présente sous forme du sel de sodium ou de benzathine et représente de 5 à 20 % en p/p de la formulation.

4. Utilisation suivant l'une quelconque des revendications 1 à 3, caractérisée en ce que l'huile acceptable du point de vue vétérinaire est une huile minérale.

5. Utilisation suivant l'une quelconque des revendications précédentes, caractérisée en ce que la formulation comprend de plus un ou plusieurs agents atténuant la douleur et/ou agents anti-inflammatoires.

6. Utilisation suivant la revendication 5, caractérisée en ce que la formulation comprend jusqu'à 0,5 % de prednisolone.

7. Utilisation suivant l'une quelconque des revendications précédentes, caractérisée en ce que les pénicillines sont présentes sous la forme de particules finement divisées, dont au moins 65 % en poids ont une dimension de l'ordre de 0 à 5 μ, en suspension dans un véhicule huileux hydrophobe qui comprend une huile et un agent gélifiant.

8. Utilisation suivant la revendication 7, caractérisée en ce qu'au moins 94 % des particules ont une dimension de l'ordre de 0 à 13 μ.

9. Utilisation suivant la revendication 7 ou la revendication 8, caractérisée en ce que les particules sont préparées par traitement dans un broyeur à billes.

10. Utilisation suivant l'une quelconque des revendications précédentes, caractérisée en ce que la formulation est sous forme de dosage unitaire ne contenant pas plus de 250 mg de pénicilline par dose unitaire.